# EUROPÄISCHE PATENTANMELDUNG

(11) **EP 3 922 636 A1**
(43) Veröffentlichungstag der Anmeldung: **15.12.2021**
(21) Anmeldenummer: 20178639.9
(22) Anmeldetag: 08.06.2020
(51) Int. Cl.: C07F 7/00, B01J 31/02, C08G 18/00

(54) **HERSTELLUNGSPROZESS FÜR AMINOALKOHOLATE**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die Erfindung betrifft ein Verfahren zur Herstellung mindestens eines Metallaminoalkoholats, mindestens umfassend die folgenden Schritte (A) Bereitstellen mindestens eines Aminoalkohols, (B) Zugabe mindestens einer basischen Verbindung zu dem in Schritt (A) bereitgestellten mindestens einen Aminoalkohol, um mindestens ein entsprechendes Aminoalkoholat zu erhalten, und (C) Zugabe mindestens eines Metallhalogenids zu der in Schritt (C) erhaltenen Mischung, um mindestens ein entsprechendes Metallaminoalkoholat zu erhalten, wobei in Schritt (C) das mindestens eine Metallhalogenid als Lösung in einem protischen Lösungsmittel mit einer Konzentration von 2,0 bis 35,0 Gew.-% zugegeben wird, eine Lösung enthaltend mindestens ein entsprechend hergestelltes Metallalkoholat, die Verwendung der Lösung zur Herstellung einer Zusammensetzung, eine entsprechende Zusammensetzung, die Verwendung dieser Zusammensetzung zur Herstellung von ein- oder mehrschichtigen Lackaufbauten, ein Verfahren zur ein- oder mehrschichtigen Beschichtung eines Substrates mit einem Lackaufbau, und ein entsprechend beschichtetes Substrat.

## Beschreibung

Die Erfindung betrifft ein Verfahren zur Herstellung mindestens eines Metallaminoalkoholats, mindestens umfassend die folgenden Schritte (A) Bereitstellen mindestens eines Aminoalkohols, (B) Zugabe mindestens einer basischen Verbindung zu dem in Schritt (A) bereitgestellten mindestens einen Aminoalkohol, um mindestens ein entsprechendes Aminoalkoholat zu erhalten, und (C) Zugabe mindestens eines Metallhalogenids zu der in Schritt (C) erhaltenen Mischung, um das entsprechende mindestens eine Metallaminoalkoholat zu erhalten, wobei in Schritt (C) das mindestens eine Metallhalogenid als Lösung in einem protischen Lösungsmittel mit einer Konzentration von 2,0 bis 35,0 Gew.-% zugegeben wird. Die vorliegende Erfindung betrifft des Weiteren eine Lösung enthaltend mindestens ein entsprechend hergestelltes Metallalkoholat, die Verwendung der Lösung zur Herstellung einer Zusammensetzung, eine entsprechende Zusammensetzung, die Verwendung dieser Zusammensetzung zur Herstellung von ein- oder mehrschichtigen Lackaufbauten, ein Verfahren zur ein- oder mehrschichtigen Beschichtung eines Substrates mit einem Lackaufbau, und ein entsprechend beschichtetes Substrat.

Verfahren zur Herstellung von Metallaminoalkoholaten, insbesondere von Sn(II)- oder Sn(IV)-alkoholaten, und deren Verwendung als thermolatente Katalysatoren bei der Herstellung von Polyurethanbeschichtungen sind dem Fachmann an sich bekannt.

DE 10 2010 012 237 A offenbart ein Verfahren zur Herstellung von Zinn(II)- oder Zinn(IV)-Alkoxiden durch Umsetzung von elementarem Zinnpulver unter Ausschluss von Sauerstoff und Wasser mit Alkoholen, um die entsprechenden Zinn(II)-Alkoholate zu erhalten. Wird diese Reaktion in Gegenwart von Sauerstoff und Wasser durchgeführt, so werden die entsprechenden Zinn(IV)-Verbindungen erhalten.

WO 2014/131750 offenbart ein Verfahren zur Herstellung von Zinn(II)- oder Zinn(IV)-Alkoholaten durch Reaktion von elementarem Zinn mit den entsprechenden Alkoholen in Gegenwart von bestimmten Aktivierungsreagenzien, beispielsweise DMF, DMSO, Sulfolan, Amin-N-Oxiden oder Pyridin-N-Oxid.

WO 09/132784 offenbart ebenfalls Zinn(IV)-Katalysatoren für die Herstellung von Polyisocyanat-Polyadditionsverbindungen. Diese Zinn(IV)-Verbindungen werden erhalten, indem entsprechende Zinn(II)-Verbindungen oxidiert werden oder Zinn(IV)-Halogenide mit Aminoalkoholen umgesetzt werden.

WO 2011/051465 offenbart ein Verfahren zur Herstellung von Metallaminoalkoholaten durch Reaktion von Metallhalogeniden mit Alkali-aminoalkoholaten in Gegenwart einer Base.

In dem Dokument WO 2011/051247 werden Zinn(IV)-aminoalkoholate als Katalysatoren zu Herstellung von Polyurethan-Beschichtungen offenbart.

Entsprechende Metallverbindungen, insbesondere Sn-enthaltende Verbindungen, werden als latente, insbesondere thermolatente, Katalysatoren zur Herstellung von ein- oder mehrschichtigen Lackaufbauten eingesetzt. Dazu liegen die Katalysatoren bevorzugt in unpolaren Lösungsmitteln, beispielsweise Butylacetat, vor. Diese Lösungen weisen an sich oft eine leichte Gelbverfärbung auf, die sich bei Lagerung und/oder insbesondere bei Kontakt mit Lackhärtern auf Polyisocyanat-Basis noch verstärken kann. Entsprechende Zubereitungen sind für qualitativ hochwertige Anwendungen, beispielsweise KFZ-Lackierungen, oft nicht geeignet.

Aufgabe der vorliegenden Erfindung war es daher, ein Verfahren zur Herstellung von Metallaminoalkoholaten, insbesondere von Zinn(IV)-aminoalkoholaten, zur Verfügung zu stellen, welches die entsprechenden Verbindungen in hoher Ausbeute zugänglich macht. Insbesondere soll das erfindungsgemäß hergestellte Metallaminoalkoholat, insbesondere Zinn(IV)-aminoalkoholat, beispielsweise zur Verwendung als thermolatenter Katalysator, bereitgestellt werden und zum einen nach der Herstellung keine Verfärbung, insbesondere keine gelbe oder orange Verfärbung, aufweisen, und des Weiteren auch nach Lagerung keine Verfärbung, insbesondere keine gelbe oder orange Verfärbung, bilden, so dass die genannte Verbindung und eine entsprechende Lösung effizient und in gleichbleibend hoher Qualität erhalten werden können, so dass sie auch für qualitativ anspruchsvolle Anwendungen, beispielsweise KFZ-Lackierungen, verlässlich geeignet sind.

Diese Aufgaben werden gelöst durch das erfindungsgemäße Verfahren zur Herstellung mindestens eines Metallaminoalkoholats, mindestens umfassend die folgenden Schritte:
(A) Bereitstellen mindestens eines Aminoalkohols,
(B) Zugabe mindestens einer basischen Verbindung zu dem in Schritt (A) bereitgestellten mindestens einen Aminoalkohol, um mindestens ein entsprechendes Aminoalkoholat zu erhalten, und
(C) Zugabe mindestens eines Metallhalogenids zu der in Schritt (B) erhaltenen Mischung, um das entsprechende mindestens eine Metallaminoalkoholat zu erhalten,
wobei in Schritt (C) das mindestens eine Metallhalogenid als Lösung in einem protischen Lösungsmittel mit einer Konzentration von 2,0 bis 35,0 Gew.-% zugegeben wird.

Die Aufgaben werden des Weiteren gelöst durch die erfindungsgemäße Lösung enthaltend mindestens ein Metallaminoalkoholat, hergestellt nach dem erfindungsgemäßen Verfahren, durch die Verwendung der erfindungsgemäßen Lösung zur Herstellung einer Zusammensetzung enthaltend das mindestens eine Metallaminoalkoholat, mindestens ein Lösungsmittel ausgewählt aus der Gruppe bestehend aus Estern, bevorzugt Butylacetat, insbesondere n-Butylacetat, Ethylacetat, Ethylenglykoldiacetat, 2-Methoxypropylacetat oder Mischungen davon, aromatischen Lösungsmitteln, bevorzugt Benzol, Toluol, Xylole oder Mischungen davon, Lactonen, bevorzugt Butyrolacton oder Mischungen davon, Carbonaten, bevorzugt Diethylcarbonat, Propylencarbonat, Ethylencarbonat oder Mischungen davon,, mindestens ein Polyisocyanat und mindestens eine NCO-reaktive Verbindung, durch die erfindungsgemäße Zusammensetzung enthaltend mindestens ein Polyisocyanat, mindestens eine NCO-reaktive Verbindung und eine erfindungsgemäße Lösung, durch die erfindungsgemäße Verwendung dieser Zusammensetzung zur Herstellung von ein- oder mehrschichtigen Lackaufbauten, durch das erfindungsgemäße Verfahren zur ein- oder mehrschichtigen Beschichtung eines Substrates mit einem Lackaufbau durch Aufbringen einer entsprechenden Zusammensetzung auf das Substrat, und durch das erfindungsgemäße Substrat, beschichtet mit einem ein- oder mehrschichtigen Lackaufbau, enthaltend eine erfindungsgemäße Zusammensetzung und/oder erhältlich durch das erfindungsgemäße Verfahren, insbesondere wobei das Substrat eine Karosserie, bevorzugt von einem Fahrzeug, insbesondere von einem Landfahrzeug, Luftfahrzeug oder Wasserfahrzeug, oder ein Teil davon, ist.

Die einzelnen Verfahrensschritte des erfindungsgemäßen Verfahrens werden im Folgenden detailliert beschrieben.

Schritt (A) des erfindungsgemäßen Verfahrens umfasst das Bereitstellen mindestens eines Aminoalkohols.

Im Allgemeinen kann in Schritt (A) des erfindungsgemäßen Verfahrens jeder dem Fachmann bekannte Aminoalkohol bereitgestellt werden. Im Rahmen der vorliegenden Erfindung steht der Begriff "Aminoalkohol" für ein organisches Molekül, welches mindestens eine Aminofunktion und mindestens eine Hydroxyfunktion aufweist. In einer bevorzugten Ausführungsform der vorliegenden Erfindung beinhaltet der in Schritt bereitgestellte mindestens eine Aminoalkohol mindestens eine Aminofunktion, bevorzugt eine sekundäre oder tertiäre, besonders bevorzugt eine tertiäre, Aminofunktion und mindestens zwei, bevorzugt zwei, Hydroxyfunktionen.

Weiter bevorzugt entspricht der in Schritt (A) des erfindungsgemäßen Verfahrens bereitgestellte mindestens eine Aminoalkohol der allgemeinen Formel (I) oder (II)

HO-X-D-Y-OH (I)

HO-X-D-Z-D-Y-OH (II),

wobei gilt:
D steht für -O-, -S- oder -N(R1)-, wobei mindestens ein vorliegendes D -N(R1)- bedeutet,
wobei R1 für einen gesättigten oder ungesättigten, linearen oder verzweigten, aliphatischen oder cycloaliphatischen oder einen gegebenenfalls substituierten, aromatischen oder araliphatischen Rest mit bis zu 20 Kohlenstoffatomen steht, der gegebenenfalls Heteroatome aus der Reihe Sauerstoff, Schwefel, Stickstoff enthalten kann, oder für Wasserstoff steht,
X, Y und Z stehen für gleiche oder unterschiedliche Reste ausgewählt aus Alkylenresten der Formeln -C(R2)(R3)-, -C(R2)(R3)-C(R4)(R5)- oder -C(R2)(R3)-C(R4)(R5)-C(R6)(R7)- oder ortho-Arylenresten der Formeln 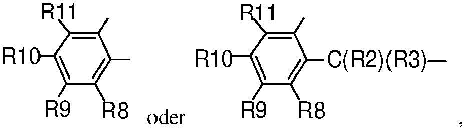
wobei R2 bis R11 unabhängig voneinander für gesättigte oder ungesättigte, lineare oder verzweigte, aliphatische oder cycloaliphatische oder gegebenenfalls substituierte, aromatische oder araliphatische Reste mit bis zu 20 Kohlenstoffatomen stehen, die gegebenenfalls Heteroatome aus der Reihe Sauerstoff, Schwefel, Stickstoff enthalten können, oder für Wasserstoff stehen;
Bevorzugt handelt es sich bei X, Y und Z um die Alkylenreste -C(R2)(R3)-, -C(R2)(R3)-C(R4)(R5)- oder den ortho-Arylenrest 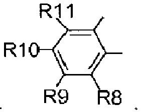
Bevorzugt handelt es sich bei R2 bis R7 um Wasserstoff oder Alkyl-, Aralkyl-, Alkaryl- oder Arylreste mit bis zu 20 C-Atomen, besonders bevorzugt um Wasserstoff oder Alkyl-, Aralkyl-, Alkaryl- oder Arylreste mit bis zu 8 C-Atomen, ganz besonders bevorzugt um Wasserstoff oder Alkylreste mit bis zu 8 C-Atomen, noch weiter bevorzugt um Wasserstoff oder Methyl.

Bevorzugt handelt es sich bei R8 bis R11 um Wasserstoff oder Alkylreste mit bis zu 8 C-Atomen, besonders bevorzugt um Wasserstoff oder Methyl.

Die Einheiten HO-X und HO-Y stehen bevorzugt für -CH₂CH₂OH, -CH₂CH(Me)OH,-CH(Me)CH₂OH, -CH₂C(Me)₂OH, -C(Me)₂CH₂OH oder -CH₂C(=O)OH.

Die Einheit HO-X-D-Y-OH steht bevorzugt für: HN[CH₂CH₂OH]₂, HN[CH₂CH(Me)OH]₂, HN[CH₂CH(Me)OH] [CH(Me)CH₂OH], HN[CH₂C(Me)₂OH]₂, HN[CH₂C(Me)₂OH][C(Me)₂CH₂OH], HN[CH₂C(=O)OH]₂, MeN[CH₂CH₂OH]₂, MeN[CH₂CH(Me)OH]₂, MeN[CH₂CH(Me)OH][CH(Me)CH₂OH], MeN[CH₂C(Me)₂OH]₂, MeN[CH₂C(Me)₂OH][C(Me)₂CH₂OH], MeN[CH₂C(=O)OH]₂, EtN[CH₂CH₂OH]₂, EtN[CH₂CH(Me)OH]₂, EtN[CH₂CH(Me)OH][CH(Me)CH₂OH], EtN[CH₂C(Me)₂OH]₂, EtN[CH₂C(Me)₂OH][C(Me)₂CH₂OH], EtN[CH₂C(=O)OH]₂, PrN[CH₂CH₂OH]₂, PrN[CH₂CH(Me)OH]₂, PrN[CH₂CH(Me)OH][CH(Me)CH₂OH], PrN[CH₂C(Me)₂OH]₂, PrN[CH₂C(Me)₂OH][C(Me)₂CH₂OH], PrN[CH₂C(=O)OH]₂, BuN[CH₂CH₂OH]₂, BuN[CH₂CH(Me)OH]₂, BuN[CH₂CH(Me)OH] [CH(Me)CH₂OH], PenN[CH₂C(Me)₂OH]₂, PenN[CH₂C(Me)₂OH][C(Me)₂CH₂OH], PenN[CH₂C(=O)OH]₂, PenN[CH₂CH₂OH]₂, PenN[CH₂CH(Me)OH]₂, PenN[CH₂CH(Me)OH][CH(Me)CH₂OH], PenN[CH₂C(Me)₂OH]₂, PenN[CH₂C(Me)₂OH][C(Me)₂CH₂OH], PenN[CH₂C(=O)OH]₂, HexN[CH₂CH₂OH]₂, HexN[CH₂CH(Me)OH]₂, HexN[CH₂CH(Me)OH][CH(Me)CH₂OH], HexN[CHC(Me)₂OH]₂, HexN[CH₂C(Me)₂OH][C(Me)₂CH₂OH], HexN[CH₂C(=O)OH]₂, OctN[CH₂CH₂OH]₂, OctN[CH₂CH(Me)OH]₂, OctN[CH₂CH(Me)OH][CH(Me)CH₂OH], OctN[CH₂C(Me)₂OH]₂, OctN[CH₂C(Me)₂OH][C(Me)₂CH₂OH], OctN[CH₂C(=O)OH]₂, wobei Pr, Bu, Pen, Hex und Oct für alle isomeren Propyl-, Butyl-, Pentyl- sowie Octylreste stehen können, PhN[CH₂CH₂OH]₂, PhN[CH₂CH(Me)OH]₂, PhN[CH₂CH(Me)OH][CH(Me)CH₂OH], PhN[CH₂C(Me)₂OH]₂, PhN[CH₂C(Me)₂OH][C(Me)₂CH₂OH] oder PhN[CH₂C(=O)OH]₂.

Ganz besonders bevorzugt ist der mindestens eine Aminoalkohol ausgewählt aus der Gruppe bestehend aus N-Cyclopentyl-diethanolamin, N-Cyclohexyl-diethanolamin, N-Cyclopentyl-diisopropanolamin, N-Cyclohexyl-diisopropanolamin, N-Butyldiethanolamin, N-Butyldiisopropanolamin, N-Methyl-N,N-bis(2-hydroxybutyl)amin, N-Butyl-N,N-bis(2-hydroxybutyl)amin, N-Cyclohexyl-N,N-bis(2-hydroxybutyl)amin und Mischungen davon.

Verfahren zur Herstellung der Aminoalkohole sind dem Fachmann an sich bekannt, beispielsweise durch Umsetzung der entsprechenden Amine mit Oxiranen.

"Bereitstellen" bedeutet im Rahmen der vorliegenden Erfindung im Allgemeinen, dass der mindestens eine Aminoalkohol so vorgelegt wird, dass die in Schritt (B) des erfindungsgemäßen Verfahrens vorgesehene Umsetzung stattfinden kann. In einer Ausführungsform des erfindungsgemäßen Verfahrens wird der mindestens eine Aminoalkohol in Schritt (A) in Substanz, d.h. ohne Lösemittel, vorgelegt. In einer bevorzugten Ausführungsform des erfindungsgemäßen Verfahrens wird der mindestens eine Aminoalkohol in Schritt (A) in mindestens einem Lösemittel gelöst bereitgestellt.

Im Allgemeinen kann der mindestens eine Aminoalkohol ohne Lösemittel oder in jedem dem Fachmann als geeignet erscheinenden Lösemittel oder Lösemittelgemisch bereitgestellt werden. Bevorzugt wird der mindestens eine Aminoalkohol in Schritt (A) in mindestens einem protischen Lösemittel gelöst bereitgestellt. Im Rahmen der vorliegenden Erfindung bedeutet "protisches Lösemittel", dass das verwendete Lösemittel mindestens ein durch eine Base abspaltbares Proton aufweist, insbesondere in Form mindestens einer Hydroxyfunktion.

Erfindungsgemäß bevorzugte protische Lösemittel sind daher Alkohole, weiter bevorzugt primäre, sekundäre oder tertiäre Alkohole. Daher wird der mindestens eine Aminoalkohol in Schritt (A) des erfindungsgemäßen Verfahrens bevorzugt in einem Alkohol gelöst bereitgestellt. Das erfindungsgemäß eingesetzte Lösemittel ist dabei bevorzugt ausgewählt aus der Gruppe bestehend aus Methanol, Ethanol, Propanol, insbesondere iso-Propanol, und Mischungen davon. Ganz besonders bevorzugt wird erfindungsgemäß Methanol eingesetzt.

Die vorliegende Erfindung betrifft daher bevorzugt das erfindungsgemäße Verfahren, wobei das mindestens eine protische Lösungsmittel ausgewählt ist aus der Gruppe bestehend aus Alkoholen, bevorzugt Methanol, Ethanol, Propanol, insbesondere iso-Propanol, und Mischungen davon.

Der mindestens eine Aminoalkohol kann in Schritt (A) des erfindungsgemäßen Verfahren in mindestens einem protischen Lösemittel in jeder dem Fachmann als geeignet erscheinenden Konzentration vorliegen. Bevorzugt beträgt die Konzentration des mindestens einen Aminoalkohols in Schritt (A) des erfindungsgemäßen Verfahren in dem mindestens einen protischen Lösemittel 10 bis 99 Gew.-%. Der mindestens eine Aminoalkohol kann in Schritt (A) des erfindungsgemäßen Verfahrens auch ohne, bevorzugt protisches, Lösemittel, d.h. in Substanz, zugegeben werden.

Der mindestens eine Aminoalkohol kann in Schritt (A) des erfindungsgemäßen Verfahrens im Allgemeinen in jedem dem Fachmann bekannten Gefäß bzw. Reaktor bereitgestellt werden. Beispiele für geeignete Gefäße bzw. Reaktoren sind Rührreaktor, Umlaufreaktoren, Plugflow-Reaktoren mit Einbauten, z.B. statische Mischer, zur Mischung oder Dosierung der Komponenten, z.B. Düsen, Membranen.

Schritt (A) des erfindungsgemäßen Verfahrens kann im Allgemeinen bei jeder dem Fachmann als geeignet erscheinenden Temperatur erfolgen. Bevorzugt erfolgt Schritt (A) des erfindungsgemäßen Verfahrens bei einer Temperatur von -20 bis 150 °C, besonders bevorzugt 0 bis 30 °C.

Schritt (A) des erfindungsgemäßen Verfahrens kann im Allgemeinen bei jedem dem Fachmann als geeignet erscheinenden Druck erfolgen. Bevorzugt erfolgt Schritt (A) des erfindungsgemäßen Verfahrens bei einem Druck von 0 bis 10 bar (a), besonders bevorzugt bei 0, 1 bis 1 bar (a), insbesondere bei Atmosphärendruck.

Schritt (A) des erfindungsgemäßen Verfahrens kann unter Schutzgas, beispielsweise Stickstoff oder Argon, durchgeführt werden. Bevorzugt wird Schritt (A) des erfindungsgemäßen Verfahrens unter Schutzgas durchgeführt.

Im Allgemeinen können nach Schritt (A) und vor Schritt (B) des erfindungsgemäßen Verfahrens weitere dem Fachmann bekannte Verfahrensschritte erfolgen, beispielsweise Temperierung, Filtration oder Konditionierung der Lösung, beispielsweise Inertisierung, Trocknung, Aktivierung durch dritte Komponenten, Stabilisierung. In einer bevorzugten Ausführungsform erfolgt erfindungsgemäß nach Schritt (A) Schritt (B) ohne weitere Zwischenschritte.

Schritt (B) des erfindungsgemäßen Verfahrens umfasst die Zugabe mindestens einer basischen Verbindung zu dem in Schritt (A) bereitgestellten mindestens einen Aminoalkohol, um mindestens ein entsprechendes Aminoalkoholat zu erhalten.

In Schritt (B) des erfindungsgemäßen Verfahrens kann im Allgemeinen jede dem Fachmann bekannte mindestens eine basische Verbindung eingesetzt werden, die in der Lage ist, den vorliegenden mindestens einen Aminoalkohol in das entsprechende Aminoalkoholat zu überführen. Erfindungsgemäß werden bevorzugt Aminoalkohole eingesetzt, die mindestens zwei Hydroxyfunktionen aufweisen. Erfindungsgemäß wird daher in Schritt (B) bevorzugt mindestens eine basische Verbindung eingesetzt, die in der Lage ist, aus den mindestens zwei Hydroxyfunktionen die Protonen zu abstrahieren.

Die in Schritt (B) des erfindungsgemäßen Verfahrens eingesetzte mindestens eine basische Verbindung ist bevorzugt ausgewählt aus der Gruppe bestehend aus Ammoniak, in wässriger und/oder alkoholischer Lösung oder als flüssiger Ammoniak, ein-, zwei- oder dreiwertigen Salzen von Alkoholen, Alkali- oder Erdalkalihydroxiden, Ammonium-, Alkali- oder Erdalkalicarbonaten und Mischungen davon.

In einer bevorzugten Ausführungsform der vorliegenden Erfindung wird als mindestens eine basische Verbindung mindestens ein ein-, zwei- oder dreiwertiges Salz von mindestens einem Alkohol eingesetzt. Bevorzugte einwertige Salze sind Alkalimetallsalze des mindestens einen Alkohols, so genannte Alkalimetallalkoholate. Bevorzugte Alkalimetalle sind Lithium, Natrium, Kalium, Caesium oder Mischungen davon. Bevorzugte zweiwertige Salze sind erfindungsgemäß Erdalkalimetallsalze des mindestens einen Alkohols, so genannte Erdalkalimetallalkoholate. Bevorzugte Erdalkalimetalle sind Magnesium, Calcium, Barium oder Mischungen davon. Erfindungsgemäß geeignete Alkoholate basieren bevorzugt auf Methanol, Ethanol oder Mischungen davon. Erfindungsgemäß besonders bevorzugt werden als mindestens eine basische Verbindung daher Alkalimetallmethanolate, insbesondere Natriummethanolat, eingesetzt.

Die vorliegende Erfindung betrifft bevorzugt das erfindungsgemäße Verfahren, wobei die mindestens eine basische Verbindung Ammoniak, ein Alkali- oder Erdalkalisalz des Anions des mindestens einen protischen Lösungsmittels, bevorzugt ein Natriumalkoholat, besonders bevorzugt ausgewählt aus der Gruppe bestehend aus Natriummethanolat, Natriumethanolat, Natriumpropanolat und Mischungen davon, ist.

Die in Schritt (B) des erfindungsgemäßen Verfahrens eingesetzte mindestens eine basische Verbindung wird bevorzugt in einer Menge eingesetzt, die ausreicht, die in dem mindestens einen Aminoalkohol vorliegenden Protonen zu entfernen. Das molare Verhältnis von in der mindestens einen basischen Verbindung vorliegenden basischen Gruppe zu den in der dem mindestens einen Aminoalkohol vorliegenden Proton beträgt daher bevorzugt 0,8 bis 1,2, besonders bevorzugt 0,9 bis 1,1, besonders bevorzugt 0,95 bis 1,05.

Verfahren zur Herstellung der erfindungsgemäß bevorzugten Metallalkoholate sind dem Fachmann an sich bekannt, beispielsweise durch Umsetzung von elementarem Alkalimetall oder Erdalkalimetall, Alkalimetall- oder Erdalkalimetallmischungen oder Alkalimetall oder Erdalkalimetall enthaltenden Legierungen wie beispielsweise Amalgamen mit dem entsprechenden Alkohol unter gleichzeitiger Bildung von Wasserstoff.

In einer weiter bevorzugten Ausführungsform der vorliegenden Erfindung wird die in Schritt (B) des erfindungsgemäßen Verfahrens eingesetzte mindestens eine basische Verbindung, in mindestens einem Lösemittel, bevorzugt in mindestens einem protischen Lösemittel, zugegeben.

Erfindungsgemäß in Schritt (B) bevorzugte protische Lösemittel sind Alkohole, weiter bevorzugt primäre, sekundäre oder tertiäre Alkohole. Daher wird die mindestens eine basische Verbindung in Schritt (B) des erfindungsgemäßen Verfahrens bevorzugt gelöst in einem Alkohol bereitgestellt.

Das erfindungsgemäße eingesetzte Lösemittel ist dabei bevorzugt ausgewählt aus der Gruppe bestehend aus Methanol, Ethanol, Propanol, insbesondere iso-Propanol, und Mischungen davon. Ganz besonders bevorzugt wird erfindungsgemäß Methanol eingesetzt.

Erfindungsgemäß bevorzugt wird die mindestens eine basische Verbindung in dem gleichen Lösungsmittel gelöst eingesetzt, in dem auch der mindestens eine Aminoalkohol in Schritt (A) des erfindungsgemäßen Verfahrens bereitgestellt wird. Ganz besonders bevorzugt wird in Schritt (A) und in Schritt (B) Methanol eingesetzt.

Die mindestens eine basische Verbindung kann in Schritt (B) des erfindungsgemäßen Verfahrens in mindestens einem Lösemittel oder Lösemittelgemisch, bevorzugt in einem protischen Lösemittel, in jeder dem Fachmann als geeignet erscheinenden Konzentration vorliegen. Bevorzugt beträgt die Konzentration der mindestens einen basischen Verbindung in dem mindestens einem Lösemittel oder Lösemittelgemisch 0,1 bis 100 Gew.-%, besonders bevorzugt 0,1 bis 35 Gew.-%.

Die Zugabe der mindestens einen basischen Verbindung in Schritt (B) des erfindungsgemäßen Verfahrens kann im Allgemeinen in jedem dem Fachmann bekannten Gefäß bzw. Reaktor erfolgen. Bevorzugt erfolgt die Zugabe gemäß Schritt (B) des erfindungsgemäßen Verfahrens in dem Gefäß oder Reaktor, in dem Schritt (A) erfolgt ist. Die Zugabe an sich kann durch dem Fachmann bekannte Vorrichtungen, beispielsweise Tropftrichter, erfolgen.

Schritt (B) des erfindungsgemäßen Verfahrens kann im Allgemeinen bei jeder dem Fachmann als geeignet erscheinenden Temperatur erfolgen. Bevorzugt erfolgt Schritt (B) des erfindungsgemäßen Verfahrens bei einer Temperatur von -30 bis 60 °C, besonders bevorzug -20 bis 30 °C.

Schritt (B) des erfindungsgemäßen Verfahrens kann im Allgemeinen bei jedem dem Fachmann als geeignet erscheinenden Druck erfolgen. Bevorzugt erfolgt Schritt (B) des erfindungsgemäßen Verfahrens bei einem Druck von 0 bis 2 bar (a), besonders bevorzugt bei 0,5 bis 1 bar (a).

Schritt (B) des erfindungsgemäßen Verfahrens kann unter Schutzgas, beispielsweise Stickstoff oder Argon, durchgeführt werden. Bevorzugt wird Schritt (B) des erfindungsgemäßen Verfahrens unter Schutzgas durchgeführt.

Nach Schritt (B) des erfindungsgemäßen Verfahrens liegt der in Schritt (A) bereitgestellte mindestens eine Aminoalkohol in deprotonierter, bevorzugt in vollständig deprotonierter, Form, d.h. als Aminoalkoholat, vor.

Im Allgemeinen können nach Schritt (B) und vor Schritt (C) des erfindungsgemäßen Verfahrens weitere dem Fachmann bekannte Verfahrensschritte erfolgen, beispielsweise Aufkonzentrierung, Konditionierung, beispielsweise Abtrennung von gelösten Inertgasen, Aktivierungen von eingesetzten Komponenten, Filtrationen und/oder Entfärbungen. In einer bevorzugten Ausführungsform erfolgt erfindungsgemäß nach Schritt (B) Schritt (C) ohne weitere Zwischenschritte, wobei gegebenenfalls eine Inertisierung erfolgt.

Schritt (C) des erfindungsgemäßen Verfahrens umfasst die Zugabe mindestens eines Metallhalogenids zu der in Schritt (C) erhaltenen Mischung, um das entsprechende mindestens eine Metallaminoalkoholat zu erhalten, wobei in Schritt (C) das mindestens eine Metallhalogenid als Lösung in einem protischen Lösungsmittel mit einer Konzentration von 0,1 bis 5,0 Gew.-% zugegeben wird.

Im Allgemeinen kann in Schritt (C) des erfindungsgemäßen Verfahrens jedes dem Fachmann als geeignet erscheinende Metallhalogenid eingesetzt werden, welches mit dem in der Lösung aus Schritt (B) vorliegenden mindestens einen Aminoalkoholat zu dem erfindungsgemäß gewünschten mindestens einen Metallaminoalkoholat reagiert.

Erfindungswesentlich ist, dass in Schritt (C) das mindestens eine Metallhalogenid als Lösung in einem protischen Lösungsmittel mit einer Konzentration von 2,0 bis 35,0 Gew.-%, bevorzugt 5,0 bis 15,0 Gew.-%, jeweils bezogen auf die zugegebene Lösung, zugegeben wird. Der angewendete Druck liegt bei 0 bis 5 bar (a), bevorzugt bei 0,2 bis 0,95 bar (a), besonders bevorzugt bei 0,4 bis 0,8 bar (a).

Das in dem erfindungsgemäßen Metallaminoalkoholat vorliegende mindestens eine Metall ist bevorzugt ausgewählt aus der Gruppe bestehend aus Zinn, insbesondere Zinn-(IV), Titan, insbesondere Titan-(IV), Bismut, insbesondere Bi-(III), und Mischungen davon.

Die vorliegende Erfindung betrifft daher bevorzugt das erfindungsgemäße Verfahren, wobei das mindestens eine Metall ausgewählt ist aus der Gruppe bestehend aus Zinn, insbesondere Zinn-(IV), Titan, insbesondere Titan-(IV), Bismut, insbesondere Bi-(III), und Mischungen davon.

Besonders bevorzugt ist das in Schritt (C) des erfindungsgemäßen Verfahrens eingesetzte mindestens eine Metallhalogenid ausgewählt aus der Gruppe bestehend aus Zinn-(IV)-chlorid, Titan-(IV)-chlorid und Mischungen davon.

Das in Schritt (C) des erfindungsgemäßen Verfahrens eingesetzte mindestens eine Metallhalogenid wird bevorzugt in einer Menge eingesetzt, so dass das gesamte aus Schritt (B) vorliegende mindestens eine Aminoalkoholat umgesetzt werden kann. Das molare Verhältnis von in dem mindestens einen Aminoalkoholat vorliegenden deprotonierten Hydroxyfunktion zu den Wertigkeit des mindestens einen vorliegenden Metallkations, d.h. bevorzugt +IV, beträgt daher bevorzugt 0,8 bis 1,2, besonders bevorzugt 0,95 bis 1,05.

Schritt (C) des erfindungsgemäßen Verfahrens kann im Allgemeinen bei jeder dem Fachmann als geeignet erscheinenden Temperatur erfolgen. Bevorzugt erfolgt Schritt (C) des erfindungsgemäßen Verfahrens bei einer Temperatur von -30 bis 60 °C, besonders bevorzug -20 bis 30 °C.

Schritt (C) des erfindungsgemäßen Verfahrens kann im Allgemeinen bei jedem dem Fachmann als geeignet erscheinenden Druck erfolgen. Bevorzugt erfolgt Schritt (C) des erfindungsgemäßen Verfahrens bei einem Druck von 0 bis 2 bar (a), besonders bevorzugt bei 0,5 bis 1 bar (a).

Schritt (C) des erfindungsgemäßen Verfahrens kann unter Schutzgas, beispielsweise Stickstoff oder Argon, durchgeführt werden. Bevorzugt wird Schritt (C) des erfindungsgemäßen Verfahrens unter Schutzgas durchgeführt.

Nach Schritt (C) des erfindungsgemäßen Verfahrens wird eine Lösung des mindestens einen entsprechenden Metallaminoalkoholats in dem mindestens einen protischen Lösungsmittel erhalten.

An Schritt (C) des erfindungsgemäßen Verfahrens können sich weitere, dem Fachmann an sich bekannte, Verfahrensschritte anschließen, beispielsweise Aufarbeitung und/oder Aufreinigung. Insbesondere können gegebenenfalls vorliegende Feststoffe abfiltriert werden. Des Weiteren kann die erhaltene Lösung von dem mindestens einen protischen Lösungsmittel befreit werden, insbesondere durch Destillation. Der so erhaltene Rückstand kann dann mit einem geeigneten Lösemittel aufgenommen werden.

Dieses Lösemittel kann erfindungsgemäß ausgewählt werden aus der Gruppe bestehend aus Estern, bevorzugt Butylacetat, insbesondere n-Butylacetat, Ethylacetat, Ethylenglykoldiacetat, 2-Methoxypropylacetat oder Mischungen davon, aromatischen Lösungsmitteln, bevorzugt Benzol, Toluol, Xylole oder Mischungen davon, Lactonen, bevorzugt Butyrolacton oder Mischungen davon, Carbonaten, bevorzugt Diethylcarbonat, Propylencarbonat, Ethylencarbonat oder Mischungen davon.

Die vorliegende Erfindung betrifft des Weiteren auch eine Lösung enthaltend mindestens ein Metallalkoholat, hergestellt nach dem erfindungsgemäßen Verfahren.

Dabei kann das in der erfindungsgemäßen Lösung vorliegende mindestens eine Lösemittel ein protisches Lösemittel ausgewählt aus der Gruppe bestehend aus Methanol, Ethanol, Propanol, insbesondere iso-Propanol, und Mischungen davon sein.

Des Weiteren kann das in der erfindungsgemäßen Lösung vorliegende mindestens eine Lösemittel ausgewählt sein aus der Gruppe bestehend aus Estern, bevorzugt Butylacetat, insbesondere n-Butylacetat, Ethylacetat, Ethylenglykoldiacetat, 2-Methoxypropylacetat oder Mischungen davon, aromatischen Lösungsmitteln, bevorzugt Benzol, Toluol, Xylole oder Mischungen davon, Lactonen, bevorzugt Butyrolacton oder Mischungen davon, Carbonaten, bevorzugt Diethylcarbonat, Propylencarbonat, Ethylencarbonat oder Mischungen davon.

Die erfindungsgemäße Lösung weist bevorzugt eine Hazen-Farbzahl, gemessen spektrofotometrisch nach DIN EN ISO 6271-2:2005-03, von weniger als 500 APHA, bevorzugt weniger als 200 APHA, besonders bevorzugt weniger als 100 APHA, ganz besonders bevorzugt weniger als 80 APHA, insbesondere weniger als 60 APHA, auf. Die Hazen-Farbzahl, gemessen spektrofotometrisch nach DIN EN ISO 6271-2:2005-03 beträgt dabei bevorzugt mindestens 1 APHA.

Die vorliegende Erfindung betrifft des Weiteren die Verwendung der erfindungsgemäßen Lösung zur Herstellung einer Zusammensetzung enthaltend das mindestens eine Metallaminoalkoholat, mindestens ein Lösungsmittel ausgewählt aus der Gruppe bestehend aus Estern, bevorzugt Butylacetat, insbesondere n-Butylacetat, Ethylacetat, Ethylenglykoldiacetat, 2-Methoxypropylacetat oder Mischungen davon, aromatischen Lösungsmitteln, bevorzugt Benzol, Toluol, Xylole oder Mischungen davon, Lactonen, bevorzugt Butyrolacton oder Mischungen davon, Carbonaten, bevorzugt Diethylcarbonat, Propylencarbonat, Ethylencarbonat oder Mischungen davon, mindestens ein Polyisocyanat und mindestens eine NCO-reaktive Verbindung.

Des Weiteren betrifft die vorliegende Erfindung die Zusammensetzung enthaltend mindestens ein Polyisocyanat, mindestens eine NCO-reaktive Verbindung und eine erfindungsgemäße Lösung.

Die vorliegende Erfindung betrifft auch die Verwendung der erfindungsgemäßen Zusammensetzung zur Herstellung von ein- oder mehrschichtigen Lackaufbauten.

Die vorliegende Erfindung betrifft auch das Verfahren zur ein- oder mehrschichtigen Beschichtung eines Substrates mit einem Lackaufbau durch Aufbringen einer erfindungsgemäßen Zusammensetzung auf das Substrat.

Die vorliegende Erfindung betrifft auch das Substrat, beschichtet mit einem ein- oder mehrschichtigen Lackaufbau, enthaltend eine erfindungsgemäße Zusammensetzung und/oder erhältlich durch das erfindungsgemäße Verfahren, wobei insbesondere das Substrat eine Karosserie, bevorzugt von einem Fahrzeug, insbesondere von einem Landfahrzeug, Luftfahrzeug oder Wasserfahrzeug, oder ein Teil davon, ist.

Entsprechende ein- oder mehrschichtige Lackaufbauten, Zusammensetzungen und Verfahren zur Herstellung der entsprechenden Beschichtungen sind beispielsweise in der WO 2017/182429 A1 beschrieben.

Erfindungsgemäß geeignete Polyisocyanate sind beispielsweise beschrieben in der WO 2017/182429 A1 und bevorzugt ausgewählt aus der Gruppe bestehend aus Di- oder Triisocyanaten, wie z.B. 1,4-Butandiisocyanat, 1,5-Pentandiisocyanat (Pentamethylendiisocyanat, PDI), 1,6-Hexandiisocyanat (Hexamethylendiisocyanat, HDI), 4-Isocyanatomethyl-1,8-octandiisocyanat (Triisocyanatononan, TIN), 4,4'-Methylenbis(cyclohexylisocyanat) (H₁₂MDI), 3,5,5-Trimethyl-1-isocyanato-3-isocyanatomethylcyclohexan (Isophorondiisocyanat, IPDI), 1,3- sowie 1,4-Bis(isocyanatomethyl)cyclohexan (H₆XDI), 1,5-Naphthalindiisocyanat, Diisocyanatodiphenylmethan (2,2'-, 2,4'- und 4,4'-MDI oder Mischungen daraus), Diisocyanatomethylbenzol (2,4- und 2,6-Toluylendiisocyanat, TDI) und technische Gemische der beiden Isomeren sowie 1,3- und/oder 1,4-Bis(isocyanatomethyl)benzol (XDI), 3,3'-Dimethyl-4,4'-biphenyldiisocyanat (TODI), 1,4-Paraphenylendiisocyanat (PPDI) sowie Cyclohexyldiisocyanat (CHDI) und die aus vorgenannten, einzeln oder in Mischung erhältlichen, höhermolekularen Oligomeren mit Biuret-, Uretdion-, Isocyanurat-, Iminooxadiazindion-, Allophanat-, Urethansowie Carbodiimid/Uretonimin-Struktureinheiten, und Mischungen davon.

Bevorzugt werden Polyisocyanate auf Basis aliphatischer und cycloaliphatischer Diisocyanate eingesetzt. Die vorliegende Erfindung betrifft daher des Weiteren die erfindungsgemäße Zusammensetzung enthaltend mindestens ein Polyisocyanat, mindestens eine NCO-reaktive Verbindung und eine erfindungsgemäße Lösung, d.h. die erfindungsgemäße Lösung B.

Erfindungsgemäß geeignete NCO-reaktive Verbindungen sind beispielsweise beschrieben in der WO 2017/182429 A1 und insbesondere ausgewählt aus der Gruppe bestehend aus niedermolekularen Diolen, z.B. 1,2-Ethandiol, 1,3- bzw. 1,2-Propandiol, 1,4-Butandiol, Triolen, z.B. Glycerin, Trimethylolpropan, Tetraolen, z.B. Pentaerythrit, kurzkettigen Polyaminen, Polyhydroxyverbindungen wie Polyetherpolyolen, Polyesterpolyolen, Polyurethanpolyolen, Polysiloxanpolyolen, Polycarbonatpolyolen, Polyetherpolyaminen, Polybutadienpolyolen, Polyacrylatpolyolen, Polymethacrylatpolyolen, deren Mischpolymerisaten, und Mischungen davon.

Die Polyhydroxyverbindungen weisen bevorzugt massenmittlere Molekulargewichte Mw > 500 Dalton, gemessen mittels Gelpermeationschromatographie (GPC) gegen einen Polystyrolstandard, besonders bevorzugt zwischen 800 und 100.000 Dalton, insbesondere zwischen 1.000 und 50.000 Dalton auf.

Die Polyhydroxyverbindungen weisen bevorzugt eine OH-Zahl von 30 bis 400 mg KOH/g, insbesondere zwischen 100 und 300 KOH/g, auf. Die Hydroxylzahl (OH-Zahl) gibt an, wie viel mg Kaliumhydroxid der Essigsäure-Menge äquivalent sind, die von 1 g Substanz bei der Acetylierung gebunden wird. Die Probe wird bei der Bestimmung mit Essigsäureanhydrid-Pyridin gekocht und die entstehende Säure mit Kaliumhydroxidlösung titriert (DIN 53240-2).

Die Glasübergangstemperaturen, gemessen mit Hilfe von DSC-Messungen nach DIN-EN-ISO 1 1357-2, der Polyhydroxyverbindungen liegen bevorzugt zwischen -150 und 100 °C, besonders bevorzugt zwischen -120°C und 80°C.

### Beispiele

### Methoden und Materialien:

Die Messung der Hazen-Farbzahl erfolgte spektrofotometrisch nach DIN EN ISO 6271-2:2005-03 mit einem LICO 400-Spektrofotometer der Fa. Lange, DE.

Die eingesetzten Verbindungen wurden, falls nicht anders angegeben, im Chemikalienhandel, beispielsweise Sigma-Aldrich, Merck KGaA, Darmstadt, Deutschland oder BASF SE, Ludwigshafen, Deutschland, bezogen und lagen jeweils in einer Reinheit von mehr als 99 Gew.-% vor.

Die Identität und Reinheit der neu synthetisierten Verbindungen wurde jeweils zweifelsfrei aus den Ergebnissen NMR-spektroskopischer, gaschromatographischer sowie massenspektrometrischer Untersuchungen bestimmt.

Herstellvorschriften für die nicht kommerziell zugänglichen N-Alkyldialkanolamine, die als Liganden für die Katalysatoren in den Beispielen a), b), c), d), f), g), h) und i) zum Einsatz kamen

Allgemeine Herstellvorschrift

In einen 2 L Laborautoklaven wurde unter Stickstoffatmosphäre das jeweilige in der untenstehenden Tabelle 1 angegebene Amin gegeben. Seine eingewogene Menge wurde so bemessen, dass sie mit den eindosierten Epoxiden im molaren Verhältnis 1:2 stand und zu einer theoretischen Gesamtausbeute von jeweils 1500 g führte. Sauerstoff wurde bei Zimmertemperatur durch 4-maliges Beaufschlagen des Autoklaven mit Stickstoff bis 3 bar (a) und anschließendes Ablassen des Überdrucks auf Normaldruck entfernt. Nach Schließen des Autoklaven wurde sein Inhalt unter Rühren (800 U/min, Kreuzbalkenrührer) auf 90 °C (Reaktionstemperatur) erwärmt. Nach Erreichen der Reaktionstemperatur und einem vom jeweiligen Amin abhängigen Reaktionsstartdruck wurde das in der Tabelle angegebene Epoxid unter fortgesetztem Rühren so in den Kopfraum des Autoklaven dosiert, dass 5 bar Druck nicht überschritten wurde. In Bezug auf die eingewogene Stoffmenge an Amin wurde jeweils die doppelte Stoffmenge an Epoxid eingesetzt. Nach Ende der Epoxiddosierung schloss sich eine Nachreaktionsphase an. Diese Nachreaktionsphase wurde dann als beendet angesehen, wenn der Druckabfall unter 20 mbar / h lag. Anschließend wurde das Produkt im Vakuum (30 mbar) über einen Zeitraum von 30 min. bei 90 °C ausgeheizt.

**Tabelle 1: eingesetzte Edukte**

| **Beispiel** | **Eingesetztes Amin** | **Eingesetztes Epoxid** |
|---|---|---|
| **a)** | Cyclopentylamin | Ethylenoxid* |
| **b)** | Cyclohexylamin | Ethylenoxid* |
| **c)** | Cyclopentylamin | Methyloxiran (Propylenoxid) |
| **d)** | Cyclohexylamin | Methyloxiran (Propylenoxid) |
| **f)** | n-Butylamin | Methyloxiran (Propylenoxid) |
| **g)** | Methylamin** | Ethyloxiran (1-Butenoxid) |
| **h)** | n-Butylamin | Ethyloxiran (1-Butenoxid) |
| **i)** | Cyclohexylamin | Ethyloxiran (1-Butenoxid) |

| | | |
|---|---|---|
| * Bei den Versuchen, in denen Ethylenoxid (Oxiran) dosiert wurde, wurde vor Beginn der Epoxiddosierung 2,8 bar Stickstoffvordruck eingestellt. ** Abweichend von der in der allgemeinen Herstellvorschrift angegebenen Vorgehensweise wurde hier eine 40 %ige wässrige Lösung von Methylamin eingesetzt. Die Reaktionstemperatur betrug 75 °C. Nach Ende der Ethyloxirandosierung wurde das Wasser im Vakuum bei 80 °C über einen Zeitraum von 3 h entfernt. | | |

Die so erhaltenen Alkanolamine wurden anschließend durch Rektifikation im Vakuum gereinigt und als farblose Flüssigkeiten bzw. Öle erhalten. Brechungsindizes und Siedepunkte der erhaltenen Verbindungen sind Tabelle 2 zu entnehmen.

**Tabelle 2: Brechungsindices und Siedepunkte der Aminoalkohole**

| **Beispiel** | **Aminoalkohol** | **n_{D}²⁰** | **Kp @ 0,1 mbar [°C]** |
|---|---|---|---|
| **a)** | N-Cyclopentyl-diethanolamin | 1,4961 | 110 |
| **b)** | N-Cyclohexyl-diethanolamin | 1,4935 | 120 |
| **c)** | N-Cyclopentyl-diisopropanolamin | 1,4785 | 117 |
| **d)** | N-Cyclohexyl-diisopropanolamin | 1,4804 | 100 |
| **f)** | N-Butyl-diisopropanolamin | 1,4518 | 73 |
| **g)** | N-Methyl-N,N-bis(2-hydroxybutyl)amin | 1,4531 | 66 |
| **h)** | N-Butyl-N,N-bis(2-hydroxybutyl)amin | 1,4546 | 97 |
| **i)** | N-Cyclohexyl-N,N-bis(2-hydroxybutyl)amin | 1,4798 | 127 |

### Beispiele 1a bis 1i (nicht erfindungsgemäß, SnCl₄ unverdünnt)

In einem 11-Vierhalskolben mit Intensivkühler (Sole-gekühlt), PTFE-ummanteltem Innenthermometer (PT 100), mechanischem PTFE-ummanteltem Rührer und Tropftrichter mit Druckausgleich wurden unter Inertgasatmosphäre (trockener Stickstoff) jeweils
a) 133,0 g N-Cyclopentyl-diethanolamin,
b) 143,8 g N-Cyclohexyl-diethanolamin,
c) 154,6 g N-Cyclopentyl-diisopropanolamin,
d) 165,3 g N-Cyclohexyl-diisopropanolamin,
e) 123,8 g N-Butyldiethanolamin,
f) 145,3 g N-Butyldiisopropanolamin,
g) 134,6 g N-Methyl-N,N-bis(2-hydroxybutyl)amin,
h) 166,9 g N-Butyl-N,N-bis(2-hydroxybutyl)amin bzw.
i) 186,9 g N-Cyclohexyl-N,N-bis(2-hydroxybutyl)amin
vorgelegt und mit jeweils 276,5 g einer 30%igen methanolischen Natriummethylatlösung versetzt. Durch Zugabe weiteren Methanols wurde der Reaktorinhalt einheitlich auf ca. 600 ml eingestellt. Anschließend wurden unter Rühren und externer Solekühlung (Innentemperatur 0 bis 5 °C) jeweils 100 g frisch destilliertes Zinntetrachlorid mittels eines Tropftrichters mit Druckausgleich langsam zugetropft.

Es setzten starke Nebelbindung im Reaktor und Feststoffabscheidung in der Flüssigkeit ein. Unabhängig von Zutropf- sowie Rührgeschwindigkeit bildeten sich dabei in schwer kontrollierbarer Weise Anbackungen am Tropftrichter und mitunter auch oberhalb der Flüssigkeitsoberfläche, die auch durch kräftiges Rühren und Anlegen von Vakuum bis zum Sieden des Reaktionsgemisches nicht reproduzierbar und vollständig zerkleinert und in die flüssige Phase überführt werden konnten.

Nach Filtration, Waschen der Niederschläge mit 3 mal ca. 100 ml frischem Methanol, Einengen im Vakuum auf Druckkonstanz bei max. 30 °C Produkttemperatur und ca. 0,1 mbar wurde mit Stickstoff belüftet und jeweils mit ca. 500 ml n-Butylacetat aufgenommen, von nicht gelösten Bestandteilen abfiltriert und an einer wirksamen Kolonne bei stufenweise reduziertem Druck so lange destilliert, bis reines n-Butylacetat am Kopf anstand, d.h. kein Methanol mehr in der Vorlage vorhanden war.

Die als Destillationssumpf verbliebenen klaren, leicht verfärbten Lösungen (Hazen-Farbzahl jeweils < 200 Apha) wurden auf einheitlich 1,0 Gew.-% Zinngehalt - jeweils bestimmt mittels Röntgenfluoreszenzanalyse - eingestellt und für die Herstellung der in Beispielserie 3-1 aufgeführten Produkte verwendet.

Die Versuche 1a bis 1i wurden wiederholt, mit dem Unterschied, dass 99,5 g (Versuche 1a-1 bis li-1) bzw. 100,5 g (Versuche 1a-2 bis li-2) frisch destilliertes Zinntetrachlorid zugesetzt wurden. Der Reaktionsverlauf war optisch identisch, analytisch (NMR, Elementaranalyse, Farbbestimmung) waren praktisch keine Unterschiede bei den erhaltenen jeweils 3 Produkten gleicher chemischer Struktur zu erkennen. Auch diese Lösungen wurden für die Herstellung der in Beispielserie 3-1 aufgeführten Produkte verwendet.

### Beispiele 2a bis 2i (erfindungsgemäß, SnCl₄ verdünnt)

In einem 11-Vierhalskolben mit Intensivkühler (Sole-gekühlt), PTFE-ummanteltem Innenthermometer (PT 100), mechanischem PTFE-ummanteltem Rührer und Tropftrichter mit Druckausgleich wurden unter Inertgasatmosphäre (trockener Stickstoff) jeweils
a) 13,3 g N-Cyclopentyl-diethanolamin,
b) 14,4 g N-Cyclohexyl-diethanolamin,
c) 15,5 g N-Cyclopentyl-diisopropanolamin,
d) 16,5 g N-Cyclohexyl-diisopropanolamin,
e) 12,4 g N-Butyldiethanolamin,
f) 14,5 g N-Butyldiisopropanolamin,
g) 13,5 g N-Methyl-N,N-bis(2-hydroxybutyl)amin,
h) 16,7 g N-Butyl-N,N-bis(2-hydroxybutyl)amin bzw.
i) 18,7 g N-Cyclohexyl-N,N-bis(2-hydroxybutyl)amin
vorgelegt, mit jeweils 27,7 g einer 30%igen methanolischen Natriummethylatlösung versetzt. Durch Zugabe weiteren Methanols wurde der Reaktorinhalt einheitlich auf ca. 100 ml eingestellt.

Anschließend wurden unter Rühren und externer Solekühlung (Innentemperatur 0 bis 5 °C) jeweils 1000 g einer nahezu farblosen, klaren, flüssigen Mischung bestehend aus 100 g frisch destilliertem Zinntetrachlorid und 900 g Methanol (im weiteren Text: 10%ige methanolische SnCl₄-Lösung) mittels eines Tropftrichters mit Druckausgleich langsam zugetropft.

Es setzte keine Nebelbindung im Reaktor ein, die Feststoffabscheidung in der Flüssigkeit verlief optisch ähnlich zu der in den Versuchsserien 2. Es war in keinem Fall die Bildung von Anbackungen am Tropftrichter oder oberhalb der Flüssigkeitsoberfläche zu beobachten.
Die nach Aufarbeitung wie unter 2 beschrieben, verbliebenen klaren, nahezu farblosen Lösungen (Hazen-Farbzahl jeweils < 100 Apha) wurden auf einheitlich 1,0 Gew.-% Zinngehalt - jeweils bestimmt mittels Röntgenfluoreszenzanalyse - eingestellt und für die Herstellung der in Beispielserie 4-2 aufgeführten Produkte verwendet.

Die Versuche 2a bis 2i wurden wiederholt, mit dem Unterschied, dass 995 g (Versuche 2a-1 bis 2i-1) bzw. 1005 g (Versuche 2a-2 bis 2i-2) der 10%igen methanolischen SnCl₄-Lösung zugesetzt wurden. Der Reaktionsverlauf war optisch identisch, analytisch (NMR, Elementaranalyse, Farbbestimmung) waren praktisch keine Unterschiede bei den erhaltenen jeweils 3 Produkten gleicher chemischer Struktur zu erkennen. Auch diese Lösungen wurden für die Herstellung der in Beispielserie 3-2 aufgeführten Produkte verwendet.

### Beispielserie 3

### (Herstellung von Mischungen aus Zinn-IV-aminoalkoholaten und Polyisocyanaten; Vergleichsversuche: Serie 3-1, erfindungsgemäße Versuche: Serie 3-2)

Jeweils 90 g eines HDI-Polyisocyanates (Isocyanurattyp, Produkt Desmodur N 3300 der Fa. Covestro AG) wurden mit jeweils 6,75 g der unter Beispiel 1 bzw. Beispiel 2 erhaltenen Lösungen versetzt und anschließend durch Zugabe von n-Butylacetat auf einheitlich jeweils 100 g aufgefüllt.

Die Mischungen wurden eine Stunde bei 50 °C homogenisiert und anschließend bei 40 °C gelagert. Nachdem optisch eine deutliche Veränderung des Habitus' der Probe beobachtet wurde, erfolge eine Farb-, Viskositäts- sowie NCO-Gehalts-Bestimmung.

Bei 80% der Produkte auf Basis der in den Vergleichsversuchsserien 1a bis 1i, 1a-1 bis 1i-1 sowie 1a-2 bis 1i-2 erhaltenen Zinn-IV-aminoalkoholatlösungen erhalten wurden, lag diese maximale Lagerzeit unter 2 Monaten. Länger als 4 Monte war keine der Proben optisch einwandfrei, d.h. die Hazen-Farbzahl lag jeweils über 150 Apha.

In unspezifischer Weise traten mitunter starke Farbvertiefungen und Viskositätserhöhungen parallel ein, meist waren jedoch lediglich Farbvertiefungen (Hazen-Farbzahl >> 500 Apha) hin zu tiefgelben und orangenen Farbtönen zu beobachten.

Im Gegensatz dazu traten in der erfindungsgemäßen Serie 3-2 überaschenderweise keine derartigen Effekte bei Verwendung der nach dem erfindungsgemäßen Verfahren gemäß Beispielen 2a bis 2i/2a-1 bis 2i-1 sowie 2a-2 bis 2i-2 erhaltenen Zinn-IV-aminoalkoholatlösungen auf. Keine der Proben war bezüglich Farbe oder Viskosität/NCO-Gehalt nach 4 monatiger Lagerung auffällig.

## Patentansprüche

1. Verfahren zur Herstellung mindestens eines Metallaminoalkoholats, mindestens umfassend die folgenden Schritte:
(A) Bereitstellen mindestens eines Aminoalkohols,
(B) Zugabe mindestens einer basischen Verbindung zu dem in Schritt (A) bereitgestellten mindestens einen Aminoalkohol, um mindestens ein entsprechendes Aminoalkoholat zu erhalten, und
(C) Zugabe mindestens eines Metallhalogenids zu der in Schritt (C) erhaltenen Mischung, um das entsprechende mindestens eine Metallaminoalkoholat zu erhalten,
**dadurch gekennzeichnet, dass** in Schritt (C) das mindestens eine Metallhalogenid als Lösung in einem protischen Lösungsmittel mit einer Konzentration von 2,0 bis 35,0 Gew.-% zugegeben wird.

2. Verfahren nach Anspruch 1, **dadurch gekennzeichnet, dass** das Metall ausgewählt ist aus der Gruppe bestehend aus Zinn, insbesondere Zinn-(IV), Titan, insbesondere Titan-(IV), Bismut, insbesondere Bi-(III), und Mischungen davon.

3. Verfahren nach Anspruch 1 oder 2, **dadurch gekennzeichnet**, das die in Schritt (B) zugegebene mindestens eine basische Verbindung als Lösung in einem protischen Lösungsmittel, insbesondere einem Alkohol, bevorzugt Methanol, Ethanol, Propanol, insbesondere iso-Propanol, oder Mischungen davon, eingesetzt wird.

4. Verfahren nach einem der Ansprüche 1 bi 3, **dadurch gekennzeichnet, dass** der in Schritt (A) des erfindungsgemäßen Verfahrens bereitgestellte mindestens eine Aminoalkohol der allgemeinen Formel (I) oder (II)
HO-X-D-Y-OH (I)
HO-X-D-Z-D-Y-OH (II)
entspricht, wobei gilt:
D steht für -O-, -S- oder -N(R1)-, wobei mindestens ein vorliegendes D -N(R1)- bedeutet,
wobei R1 für einen gesättigten oder ungesättigten, linearen oder verzweigten, aliphatischen oder cycloaliphatischen oder einen gegebenenfalls substituierten, aromatischen oder araliphatischen Rest mit bis zu 20 Kohlenstoffatomen steht, der gegebenenfalls Heteroatome aus der Reihe Sauerstoff, Schwefel, Stickstoff enthalten kann, oder für Wasserstoff steht,
X, Y und Z stehen für gleiche oder unterschiedliche Reste ausgewählt aus Alkylenresten der Formeln -C(R2)(R3)-, -C(R2)(R3)-C(R4)(R5)- oder -C(R2)(R3)-C(R4)(R5)-C(R6)(R7)- oder ortho-Arylenresten der Formeln 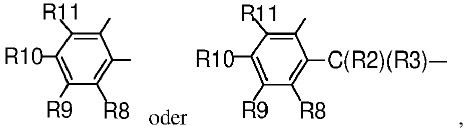
wobei R2 bis R11 unabhängig voneinander für gesättigte oder ungesättigte, lineare oder verzweigte, aliphatische oder cycloaliphatische oder gegebenenfalls substituierte, aromatische oder araliphatische Reste mit bis zu 20 Kohlenstoffatomen stehen, die gegebenenfalls Heteroatome aus der Reihe Sauerstoff, Schwefel, Stickstoff enthalten können, oder für Wasserstoff stehen.

5. Verfahren nach einem der Ansprüche 1 bis 4, **dadurch gekennzeichnet, dass** der mindestens eine Aminoalkohol ausgewählt ist aus der Gruppe bestehend aus N-Cyclopentyl-diethanolamin, N-Cyclohexyl-diethanolamin, N-Cyclopentyl-diisopropanolamin, N-Cyclohexyl-diisopropanolamin, N-Butyldiethanolamin, N-Butyldiisopropanolamin, N-Methyl-N,N-bis(2-hydroxybutyl)amin, N-Butyl-N,N-bis(2-hydroxybutyl)amin, N-Cyclohexyl-N,N-bis(2-hydroxybutyl)amin und Mischungen davon.

6. Verfahren nach einem der Ansprüche 1 bis 5, **dadurch gekennzeichnet, dass** die mindestens eine basische Verbindung Ammoniak, ein Alkali- oder Erdalkalisalz des Anions des mindestens einen protischen Lösungsmittels, bevorzugt ein Natriumalkoholat, besonders bevorzugt ausgewählt aus der Gruppe bestehend aus Natriummethanolat, Natriumethanolat, Natriumpropanolat und Mischungen davon, ist.

7. Verfahren nach einem der Ansprüche 1 bis 6, **dadurch gekennzeichnet, dass** mindestens eine protische Lösungsmittel ausgewählt ist aus der Gruppe bestehend aus Alkoholen, bevorzugt Methanol, Ethanol, Propanol, insbesondere iso-Propanol, und Mischungen davon.

8. Lösung enthaltend mindestens ein Metallalkoholat, hergestellt nach dem Verfahren gemäß einem der Ansprüche 1 bis 7.

9. Verwendung der Lösung nach Anspruch 8 zur Herstellung einer Zusammensetzung enthaltend das mindestens eine Metallaminoalkoholat, mindestens ein Lösungsmittel ausgewählt aus der Gruppe bestehend aus Estern, bevorzugt Butylacetat, insbesondere n-Butylacetat, Ethylacetat, Ethylenglykoldiacetat, 2-Methoxypropylacetat oder Mischungen davon, aromatischen Lösungsmitteln, bevorzugt Benzol, Toluol, Xylole oder Mischungen davon, Lactonen, bevorzugt Butyrolacton oder Mischungen davon, Carbonaten, bevorzugt Diethylcarbonat, Propylencarbonat, Ethylencarbonat oder Mischungen davon,, mindestens ein Polyisocyanat und mindestens eine NCO-reaktive Verbindung.

10. Zusammensetzung enthaltend mindestens ein Polyisocyanat, mindestens eine NCO-reaktive Verbindung und eine Lösung gemäß Anspruch 8.

11. Verwendung der Zusammensetzung nach Anspruch 10 zur Herstellung von ein- oder mehrschichtigen Lackaufbauten.

12. Verfahren zur ein- oder mehrschichtigen Beschichtung eines Substrates mit einem Lackaufbau durch Aufbringen einer Zusammensetzung nach Anspruch 10 auf das Substrat.

13. Substrat, beschichtet mit einem ein- oder mehrschichtigen Lackaufbau, enthaltend eine Zusammensetzung nach Anspruch 10 und/oder erhältlich durch das Verfahren nach Anspruch 12, insbesondere **dadurch gekennzeichnet, dass** das Substrat eine Karosserie, bevorzugt von einem Fahrzeug, insbesondere von einem Landfahrzeug, Luftfahrzeug oder Wasserfahrzeug, oder ein Teil davon, ist.
